# EUROPEAN PATENT SPECIFICATION

(11) **EP 4 219 321 B1**
(45) Date of publication and mention of the grant of the patent: **22.05.2024**
(21) Application number: 23152919.9
(22) Date of filing: 23.01.2023
(51) Int. Cl.: B65B 9/20, A61L 2/08, B65B 55/08, B65B 55/10, B65B 61/18, A61L 2/10

(54) **PACKAGING MACHINE AND METHOD FOR FORMING PACKAGES**
VERPACKUNGSMASCHINE UND VERFAHREN ZUM FORMEN VON VERPACKUNGEN
MACHINE D'EMBALLAGE ET PROCÉDÉ DE FORMATION D'EMBALLAGES

(30) Priority: 28.01.2022 EP 22153837
(43) Date of publication of application: 02.08.2023
(73) Proprietor: Tetra Laval Holdings & Finance S.A., 1009 Pully (CH)
(72) Inventor: ZANNINI, Antonio, 41123 Modena (IT)
(74) Representative: Tetra Pak - Patent Attorneys SE

(56) References cited:
- EP-A1- 1 232 760
- EP-A1- 3 549 613
- FR-A5- 2 055 707

## Description

### TECHNICAL FIELD

The present invention relates to a packaging machine for forming packages filled with a pourable food product from a web of packaging material, in particular a web of packaging material having a multilayer structure. The packaging machine comprises a molding apparatus for molding molded portions, in particular opening devices, onto the web of packaging material.

### BACKGROUND ART

As is known, many liquid or pourable food products, such as fruit juice, UHT (ultra-high-temperature treated) milk, wine, tomato sauce, etc., are sold in packages made of sterilized packaging material.

A typical example is the parallelepiped-shaped package for liquid or pourable food products known as Tetra Brik Aseptic (registered trademark), which is made by sealing and folding a laminated packaging material. The packaging material has a multilayer structure comprising a base layer, e.g. of paper or cardboard, covered on both sides with layers of heat-seal plastic material, e.g. polyethylene. In the case of aseptic packages for long-storage products, such as UHT milk, the packaging material also comprises a layer of oxygen-barrier material (an oxygen-barrier layer), e.g. an aluminum foil, which is superimposed on a layer of heat-seal plastic material, and is in turn covered with another layer of heat-seal plastic material forming the inner face of the package eventually contacting the food product.

Packages of this sort are normally produced on fully automatic packaging apparatuses, which advance and sterilize a web of packaging material, which is then formed into a tube and filled with the pourable pre/sterilized product under aseptic conditions before its formation into individual sealed packages. Such a packaging machine is known, for example, in EP3549613A1, which discloses a packaging machine for forming packages filled with a pourable product from a web of packaging material,the packaging machine comprises a conveying device configured to advance the web of packaging material along a web advancement path, a sterilization apparatus for sterilizing the web of packaging material and being arranged at a sterilization station along the web advancement path, an isolation chamber having an inner environment, a tube forming and sealing device configured to form a tube from the, in use, advancing web of packaging material within the inner environment at a tube formation station arranged downstream of the and to longitudinally seal the tube within the inner environment, a filling device for filling the tube with the pourable product, a package forming apparatus configured to form, to transversally seal and to transversally cut the, in use, advancing tube for forming the packages.

It is known that some types of packages also comprise respective opening devices, which allow to be manipulated for accessing the pourable product.

In order to provide the packages with the opening devices, some automatic packaging apparatuses also comprise a molding apparatus for molding the opening devices onto the web of packaging material prior to the formation of the packages.

It is furthermore known that the automatic packaging apparatus may comprise an isolation chamber within which the web of packaging material is formed and filled with the pourable product. As the web of packaging material gets into contact with the pourable product one needs to guarantee the cleanliness and/or sterility of the isolation chamber and of the web of packaging material.

Therefore, the typical automatic packaging apparatuses also comprise a sterilization apparatus arranged downstream of the molding apparatus and upstream of the isolation chamber so as to sterilize the packaging material.

It should be noted that the known sterilization apparatuses can be configured to sterilize the web of packaging material by means of chemical sterilization or by physical sterilization.

In the recent years, the Applicant has successfully established the use of the electron beam technology so as to sterilize the web of packaging material. While the sterilization of the flat web of packaging material by means of the electron beam technology is relatively easy, the situation becomes more challenging in the case of the presence of opening devices, which have been applied onto the web of packaging material. When sterilizing the web of packaging material being equipped with the opening devices, one may need to operate with rather high powers so as to obtain the desired sterility.

Thus, even though, the known packaging machine achieve excellent working results, a desire is felt in the sector to further improve the known packaging machines.

### DISCLOSURE OF INVENTION

It is an object of the present invention to provide in a straightforward and low-cost manner an improved packaging machine having a molding apparatus.

It is a further object of the present invention, to provide in a straightforward and low-cost manner an improved method for forming packages filled with a pourable food product from a web of packaging material.

According to the present invention, there is provided a packaging machine as claimed in claim 1 and a method as claimed in claim 12.

Preferred non-limiting embodiments of the packaging machine and the method are claimed in the respective dependent claims.

### BRIEF DESCRIPTION OF THE DRAWINGS

A non-limiting embodiment of the present invention will be described by way of example with reference to the accompanying drawing, in which:

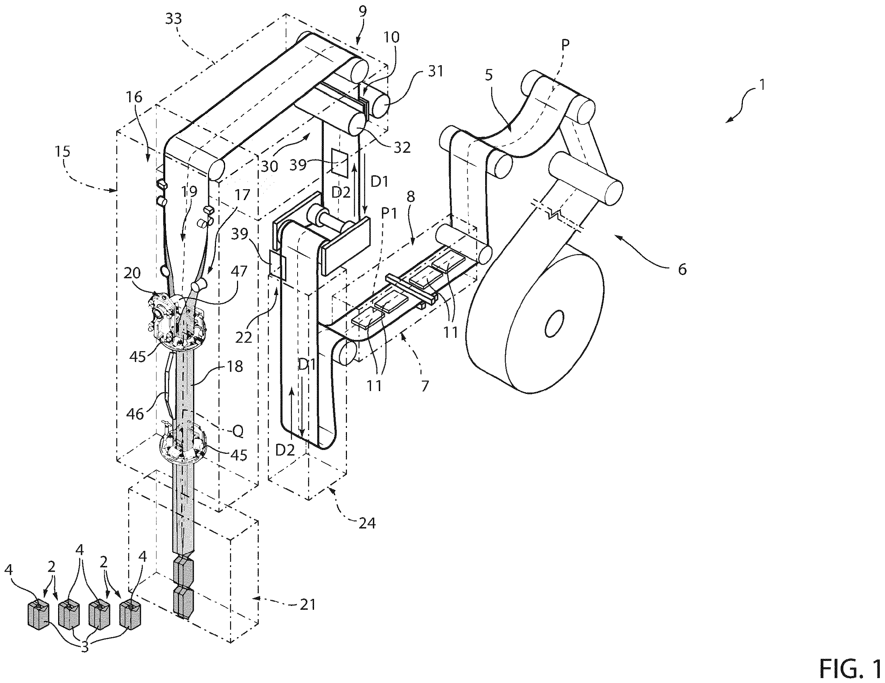

Figure 1 is a schematic view of a packaging machine according to the present invention, with parts removed for clarity.

### BEST MODES FOR CARRYING OUT THE INVENTION

Number 1 indicates as a whole a packaging machine for producing (sealed) packages 2 of a pourable food product such as pasteurized milk, fruit juice, wine, tomato sauce, emulsions, beverages with pulp etc.

Each packaging 2 may comprise a main body 3 and a molded portion 4, in particular an opening device, molded onto main body 3.

In particular, packaging machine 1 may produce packages 2, in particular the respective main bodies 3, from a web of packaging material 5.

In more detail, web of packaging material 5 may have a multilayer structure and may comprise at least a layer of fibrous material, such as e.g. a paper or cardboard layer, and at least two layers of heat-seal plastic material, e.g. polyethylene, interposing the layer of fibrous material in between one another. One of these two layers of heat-seal plastic material defines the inner face of package 2 eventually contacting the pourable product.

Moreover, web of packaging material 5 also comprises a layer of gas- and light-barrier material, e.g. aluminum foil or ethylene vinyl alcohol (EVOH) film, in particular being arranged between one of the layers of the heat-seal plastic material and the layer of fibrous material.

Additionally, web of packaging material 5 may also comprise a further layer of heat-seal plastic material being interposed between the layer of gas- and light-barrier material and the layer of fibrous material.

Web of packaging material 5 may comprise a first face and a second face, in particular the first face being the face of web of packaging material 5 defining the inner face of the formed package 2 eventually contacting the filled pourable food product.

Preferentially, web of packaging material 5 may comprise a plurality of repeat units, in particular successively arranged (and equally spaced) with respect to one another along web of packaging material 5. In particular, each repeat unit may form the basis of one respective package 2. In other words, packaging machine 1 may be configured to produce packages 2 from web of packaging material 5 such that each package 2 results from one respective repeat unit.

In particular, each repeat unit may be defined by one respective pattern present on web of packaging material 5. Even more particular, the pattern is substantially identical for all repeat units and may also differ in minor details from the other ones - minor differences may e.g. be the presence of information tags indicating a production day, a production lot, personalized information or similar.

With particular reference to Figure 1, packaging machine 1 comprises:
- a conveying device 6 configured to advance web of packaging material 5 along a web advancement path P;
- a molding apparatus 7 arranged at a molding station 8 for molding molded portions 4, in particular the opening devices, onto web of packaging material 5;
- a sterilization apparatus 9 for sterilizing web of packaging material 5 and being arranged at a sterilization station 10 downstream of molding station 8 along web advancement path P;
- an isolation chamber 15 having an inner environment 16;
- a tube forming and sealing device 17 configured to form a tube 18 from the, in use, advancing web of packaging material 5 and to longitudinally seal tube 18 within inner environment 16 at a tube formation station 19 positioned upstream of sterilization station 10 along web advancement path P;
- a filling device 20 configured to fill tube 18 with the pourable food product; and
- a package forming apparatus 21 configured to at least form and transversally seal tube 18, preferentially to also transversally cut tube 18, for forming (sealed) packages 2.

The Applicant has seen that after termination of the molding process, molded portions 4 are clean and/or sterile as a consequence of a number of factors including the high temperatures of plastic processing conditions needed during the molding process itself. Therefore, the Applicant wants to preserve (partially or in full) the cleanliness and/or sterility of molded portions 4 during advancement of web of packaging material 5 from molding station 8 to sterilization station 10.

Therefore, packaging machine1 also comprises a preserving device 22 configured to preserve the cleanliness and/or sterility of molded portions 4, in particular opening devices, in particular after molding.

In particular, preserving device 22 is configured to preserve the cleanliness and/or sterility of molded portions 4, in particular opening devices, during advancement of web of packaging material 5 between molding station 8 and sterilization station 10.

In this way, one facilitates the operation of sterilization apparatus 9.

According to some preferred non-limiting embodiments, molding apparatus 7 molds at least one respective molded portion 4, in particular one respective opening device, onto each repeat unit of web of packaging material 3.

In particular, molding apparatus 7 is configured to mold molded portions 4 from a molten polymer.

In the most preferred embodiment, molding apparatus 7 molds opening devices onto web of packaging material 3.

In particular, each opening device may be configured to allow to access and/or to outpour, in particular after a manipulation of the opening device, the pourable food product packaged within the respective package 2. In particular, the manipulation of the opening device can be reversible or irreversible.

One example of an opening device is a lid-spout assembly, which comprises a spout and a lid, which is configured to selectively close and open a pouring outlet of the spout. In order to access and/or outpour the pourable product from packages 2 being provided with such an opening device removal of the lid is required, which typically is reversible, meaning that the lid can be newly coupled to the spout.

Another example of an opening device is a strip, which can be removed from the respective package 2 for creating an outlet hole and/or for allowing to at least partially separating two portions of the respective package 2 from one another.

In further detail, molding apparatus 7 may comprise one or more molding units 11 each one configured to mold one or more molded portions 4, in particular one or more opening devices (at a time) onto web of packaging material 5, in particular onto at least one respective repeat unit.

According to some possible non-limiting embodiments, molding apparatus 7 may comprise one or more punching units configured to punch holes in web of packaging material 5, in particular prior to the operation of the one or more molding units 11. In other words, the punching unit may be arranged upstream of molding apparatuses 20 along web advancement path P.

In particular, the one or more molding units 11 may be configured to mold molded portions 4, in particular the opening devices, onto web of packaging material 5 at the holes.

Advantageously, conveying device 6 may also be configured to advance tube 18 along a tube advancement path Q towards and at least partially through package forming apparatus 21.

In further detail, conveying device 6 may also be configured to advance tube 18 and any intermediate of tube 18 along tube advancement path Q. In particular, with intermediates of tube 18 any configuration of web of packaging material 5 is meant prior to obtaining the tube structure and after folding of web of packaging material 5 has been started. In other words, the intermediates of tube 18 are a result of a gradual folding of web of packaging material 5 so as to obtain tube 18, in particular by overlapping opposite lateral edges of web of packaging material 5 with one another.

Preferentially, conveying device 6 may also be configured to advance web of packaging material 5 at and/or within molding apparatus 7 intermittently.

Even more preferentially, conveying device 6 may be configured to intermittently advance and stop web of packaging material 5, and in particular each molding unit 11 may be configured to mold the respective molded portions 4 during a stop of web of packaging material 5.

Preferentially, conveying device 6 may be configured to intermittently advance web of packaging material 5 along a molding portion P1 of path P.

Advantageously, packaging machine 1 may also comprise a first buffer unit and a second buffer unit 24 arranged respectively upstream and downstream of molding station 8 along web advancement path P.

Preferentially, the buffer capacity of the first buffer unit and second buffer unit 24 can be (dynamically) varied.

Moreover, conveying device 6 may be configured to advance web of packaging material 5 to first buffer unit and withdraw web of packaging material 5 from second buffer unit 24 in a continuous manner.

In this way, one guarantees a continuous production of packages 2 while allowing an intermittent operation of molding apparatus 7.

With particular reference to Figure 1, isolation chamber 15 is configured to protect inner environment 16 from an (hostile) outer environment.

Even more particular, isolation chamber 15 is configured to allow for forming, longitudinally sealing and filling tube 18 in a sterile inner environment 16.

Preferentially, packaging machine 1 may also comprise a conditioning unit configured to define and control the atmosphere and conditions within inner environment 16. In particular, the conditioning unit may be configured to maintain and control sterility of inner environment 16. Additionally, the conditioning unit may also be configured to control temperature and/or humidity and/or pressure.

Moreover, the conditioning unit may also be configured to maintain sterility within inner environment 16.

Preferentially, prior to operation of packaging machine 1, inner environment 16 is subjected to a sterilization process, in particular a chemical and/or physical sterilization process, so as to establish sterility of inner environment 16. In particular, a sterilization-in-place (SIP) may be executed.

With particular reference to Figure 1, sterilization apparatus 9 may comprise an irradiation device 30 configured to direct a sterilizing irradiation, in particular electromagnetic irradiation, even more particular electron beam irradiation, onto the, in use, advancing web of packaging material 5. In particular, irradiation device 30 may be configured to direct the sterilizing irradiation onto the first face and the second face of web of packaging material 5.

In further detail, irradiation device 30 may comprise one or more e-beam emitters for emitting electron beam irradiation onto the, in use, advancing web of packaging material 5, in particular onto the first face and the second face.

More precisely, irradiation device 30 may comprise a pair of e-beam emitters, in particular a first e-beam emitter 31 and a second e-beam emitter 32. Moreover, first e-beam emitter 31 and second e-beam emitter 32 are spaced apparat from one another such that, in use, the advancing web of packaging material 5 advances between first e-beam emitter 31 and second e-beam emitter 32. In particular, first e-beam emitter 31 and second e-beam emitter 32 are arranged such to direct the electron beam irradiation onto respectively the first face and the second face.

Alternatively, sterilization apparatus 9 could be configured to sterilize web of packaging material 5 by chemical sterilization.

Advantageously, sterilization apparatus 9 may comprise a housing 33 having an inlet opening for receiving web of packaging material 5 to be sterilized and an outlet opening for allowing feeding out of the sterilized web of packaging material 5.

Preferentially, the outlet opening may be adjacent to an inlet aperture of the isolation chamber 15 such that web of packaging material 5 exits, in use, sterilization apparatus 9 through the outlet opening and enters, in use, the isolation chamber 15 through the inlet aperture.

With particular reference to Figure 1, packaging machine 1 may comprise a further housing (not shown) delimiting an inner space within which molding apparatus 7 is arranged and/or within which web of packaging material 5 advances, in use, along advancement path P, in particular between at least molding station 8 and sterilization station 10.

Advantageously, preserving device 22 comprises at least one blowing unit 39 configured to generate a flow of sterile fluid, in particular of sterile gas such as sterile air, and direct the flow of sterile fluid against at least a portion of web of packaging material 5 while web of packaging material 5 advances between molding station 8 and sterilization station 10.

Preferentially, the portion of web of packaging material 5 that receives the flow of sterile fluid is intended with regard to the relative position between molding station 8 and sterilization station 10.

In particular, by providing for blowing unit 39 and the generation of the flow of sterile fluid against web of packaging material 5, one achieves that the deposition of possible contaminants on web of packaging material 3 and molded portions 4 is hampered, which again renders the sterilization by means of sterilization apparatus 9 easier. In particular, the inner space is not a sterile space, i.e. the inner space is not part of inner environment 16.

Preferentially, preserving device 22 may comprise more than one blowing unit 39, each one configured to generate a respective flow of sterile fluid, in particular of sterile gas such as sterile air, against at least a portion of web of packaging material 5 while advancing along a respective portion of web advancement path P and between molding station 8 and sterilization station 10.

More specifically, each blowing unit 39 may be positioned at a respective position different from the position of the other blowing units 39 so as to direct the respective flow of sterile fluid onto different portions of web of packaging material 5. In particular, each blowing unit 39 may be arranged at respective positions along web advancement path P between molding station 8 and sterilization station 10, in particular such that one or more blowing units 39 may be placed at upstream positions between molding station 8 and sterilization station 10 and with regard to web advancement path P and one or more other blowing units 39 may be positioned at downstream positions between molding station 8 and sterilization station 10 and with regard to web advancement path P (i.e. one or more blowing units 39 may be arranged upstream of one or more other blowing units 39) .

Preferentially, the at least one flow of sterile fluid and/or the plurality of flows of sterile fluid may be directed onto the first face and the second face of web of packaging material 5. This allows to preserve the cleanliness and/or sterility of the respective zones of molded portions 4 which are accessible from the first face and the second face, respectively.

According to some possible embodiments, each blowing unit 39 may also be configured to generate two respective flows of sterile fluid. Thereby, a first flow of sterile fluid and a second flow of sterile fluid may be directed towards the first face and the second face, respectively.

According to some preferred non-limiting embodiments, each blowing unit 39 may be configured to generate a flow of sterile fluid in a direction D1 transversal and/or opposite to an advancement direction D2 of web of packaging material 5. In particular, each advancement direction D2 is the respective direction of advancement of web of packaging material 5 at the respective portion onto which the respective flow of sterile fluid is directed onto.

It should be noted that advancement direction D2 may locally vary and accordingly, the respective directions D1 of blowing units 39 may differ from one another in dependence of the local advancement direction D2.

In a most preferential embodiment, each blowing unit 39 may be configured to generate the flow of sterile fluid such that the respective direction D1 is opposite to advancement direction D2.

It may be possible that an angle defined between directions D1 and the respective advancement directions D2 may vary. In other words, blowing units 39 may be configured such that at least one or more blowing units 39 may generate the respective flow of sterile fluid such that the angle defined with the respective advancement directions D2 may vary with regard to the respective angles defined by the respective flows of sterile fluid and the respective advancement directions D2.

Advantageously, preserving device 22 may also comprise one or more disinfection units for disinfecting at least portions, in particular molded portions 4, of web of packaging material 5 while advancing between molding station 8 and sterilization station 10.

Disinfection unit may comprise one or more UV-light emitters.

In more detail and with reference to Figure 1, tube forming and sealing device 17 may comprise at least two forming ring assemblies 45, in particular arranged within inner environment 16, being configured to gradually fold in cooperation with one another web of packaging material 5 into tube 18, in particular by overlapping the edges of web of packaging material 5 with one another.

Additionally, tube forming and sealing device 17 may comprise a sealing head 46, in particular arranged within inner environment 16 and, configured to longitudinally seal tube 18.

Additionally, filling device 20 may comprise a filling pipe 47 being configured to direct, in use, the pourable product into tube 18. In particular, filling pipe 47 may, in use, be at least partially placed within tube 18 for feeding, in use, the pourable product into tube 18.

Moreover, package forming apparatus 21 may comprise:
- a plurality of forming and sealing assemblies, each one configured to at least form (shape) tube 18, to transversally seal tube 18, and in particular to also transversally cut tube 18; and
- a conveying unit so as to advance the forming and sealing assemblies.

In particular, package forming apparatus 12 is configured to control the forming and sealing assemblies and the conveying unit such to transversally seal and cut tube 18 along equally spaced transversal cross sections.

In use, packaging machine 1 produces packages 2 of a pourable food product. In particular, packaging machine 1 forms tube 18 from web of packaging material 5, longitudinally seals tube 18, fills tube 18 with the pourable product and forms, transversally seals and transversally cuts tube 18 so as to obtain packages 2.

Prior to the formation of tube 18 from web of packaging material 5, web of packaging material 5 is sterilized by sterilization apparatus 9.

Additionally, prior to the sterilization, molding apparatus 7 molds molded portions 4, in particular the opening devices, onto web of packaging material 5, in particular the respective repeat units.

In more detail, operation of packaging machine 1 comprises the following steps:
- advancing, in particular by means of conveying device 6, web of packaging material 5 along web advancement path P;
- molding, in particular by means of molding apparatus 7, molded portions 4 onto web of packaging material 5 at molding station 8;
- sterilizing, in particular by means of sterilization apparatus 9, web of packaging material 5 at sterilization station 10;
- forming, in particular by means of tube forming and sealing device 17, tube 18 from the, in use, advancing web of packaging material 5 within inner environment 16;
- longitudinally sealing tube 18, in particular by means of tube forming and sealing device 17, within inner environment 16;
- filling tube 18, in particular by means of filling device 20, with the pourable food product;
- producing, in particular by means of package forming apparatus 21, packages 2 from tube 18 by forming, transversally sealing, and transversally cutting tube 18; and
- preserving the cleanliness and/or sterility of at least molded portions 4.

Additionally, operation of packaging machine 1 may also comprise the step of conveying tube 18 along tube advancement path Q.

Preferentially, operation of packaging machine 1 may also comprise a first step of buffering, executed by first buffer unit and a second step of buffering executed by second buffer unit 24.

During the step of preserving, the flow of sterile fluid is directed against at least a portion, in particular at least against molded portions 4, of web of packaging material 5 while web of packaging material 5 advances between molding station 8 and sterilization station 10. In particular, the flow of sterile fluid may be generated by the at least one blowing unit 39.

Preferentially, during the step of preserving, a plurality of flows of sterile fluid are generated. In particular each blowing unit 39 generates one respective flow of sterile fluid.

Moreover, each flow of sterile fluid, in particular as generated by the respective blowing units 39, may be directed against a respective portion distinct from the other portions of web of packaging material 5 while web of packaging material 5 advances along a respective portion of web advancement path P and while web of packaging material 5 advances between molding station 8 and sterilization station 10.

Preferentially, during the step of preserving, each flow of sterile fluid is such that the respective directions D1 are transversal and/or opposite to the respective advancement direction D2 of the web of packaging material 5 (in particular at the specific location) .

Additionally, each flow of sterile fluid is directed onto one of the first face and the second face or onto both the first face and the second face. Preferentially, the overall flows of sterile fluid are such that at least one flow of sterile fluid is directed onto the first face and at least one flow of sterile fluid is directed onto the second face.

According to some possible non-limiting embodiments, during the step of preserving, a sub-step of disinfecting is executed during which at least portions, in particular molded portions 4, of web of packaging material 5 are disinfected while web of packaging material 5 advances between molding station 8 and sterilization station 10.

In particular, during the sub-step of disinfecting a UV-irradiation is directed onto web of packaging material 5, in particular onto the first face and/or the second face.

In more detail, during the step of advancing, web of packaging material 5 is intermittently advanced at molding station 8 and/or within molding apparatus 7.

In particular, during the step of advancing, web of packaging material 5 is intermittently advanced between first buffer unit and second buffer unit 24.

Moreover, during the step of advancing, web of packaging material 5 is advanced along web advancement path P.

In more detail, during the step of molding, molding apparatus 7 molds molded portion 4, in particular the opening devices, onto web of packaging material 5.

In particular, during the step of molding, advancement of web of packaging material 5 at molding station 8 and/or within molding apparatus 7 is on halt.

In more detail, during the step of sterilizing, the sterilizing irradiation, in particular the electromagnetic irradiation, even more particular the electron beam irradiation, is directed onto web of packaging material 5, in particular onto the first face and the second face.

The advantages of packaging machine 1 and/or the method according to the present invention will be clear from the foregoing description.

In particular, by having preserving device 22 it is possible to maintain cleanliness and/or sterility of molded portions 4 or to at least reduce the contamination following the molding process as much as possible. This permits to facilitate the operation of the sterilization process.

Such a solution is of particular advantage when sterilization apparatus 9 relies on sterilization by means of a sterilizing irradiation. This allows to operate at lower energies, which means a longer lifetime and lower energy consumption.

Another advantage resides in that the sterilization of molded portions 4 having a more complex geometry than a flat shape is rendered more efficient and/or easier.

Clearly, changes may be made to packaging machine 1 or the method as described herein without, however, departing from the scope of protection as defined in the accompanying claims.

## Claims

1. Packaging machine (1) for forming packages (2) filled with a pourable product from a web of packaging material (5);
the packaging machine (1) comprises:
- a conveying device (6) configured to advance the web of packaging material (5) along a web advancement path (P);
- a molding apparatus (7) arranged at a molding station (8) for molding molded portions (4) onto the web of packaging material (5);
- a sterilization apparatus (9) for sterilizing the web of packaging material (5) and being arranged at a sterilization station (10) downstream of the molding station (8) along the web advancement path (P);
- an isolation chamber (15) having an inner environment (16);
- a tube forming and sealing device (17) configured to form a tube (18) from the, in use, advancing web of packaging material (5) within the inner environment (16) at a tube formation station arranged downstream of the and to longitudinally seal the tube (4) within the inner environment;
- a filling device (20) for filling the tube (18) with the pourable product;
- a package forming apparatus (21) configured to form, to transversally seal and to transversally cut the, in use, advancing tube (18) for forming the packages (2); and
- a preserving device (22) configured to preserve the cleanliness and/or sterility of at least the molded portions (4);
wherein the preserving device (22) comprises at least one blowing unit (39) configured to generate a flow of sterile fluid against at least a portion of the web of packaging material (5) while the web of packaging material (5) advances, in use, between the molding station (8) and the sterilization station (10).

2. Packaging machine according to claim 1, wherein the blowing unit (39) is configured to generate the flow of sterile fluid in a direction (D1) transversal and/or opposite to an advancement direction (D2) of the web of packaging material (5).

3. Packaging machine according to claim 1 or 2, wherein the preserving device (22) comprises more than one blowing unit (39), each one configured to generate a respective flow of sterile fluid against at least a respective portion of the web of packaging material (5) while the web of packaging material (5) advances along a respective portion of the web advancement path (P) and between the molding station (8) and the sterilization station (10).

4. Packaging machine according to any one of the preceding claims, wherein the preserving device (22) also comprises a disinfection unit for disinfecting the web of packaging material (5) while the web of packaging material (5) advances between the molding station (8) and the sterilization station (10).

5. Packaging machine according to claim 4, wherein the disinfection unit comprises a UV-light emitter.

6. Packaging machine according to any one of the preceding claims, and further comprising a housing delimiting an inner space within which the web of packaging material (5) advances, in use, when moving along the web advancement path (P).

7. Packaging machine according to claim 6, wherein the molding apparatus (7) is arranged within the housing.

8. Packaging machine according to any one of the preceding claims, wherein the sterilization apparatus (9) comprises an irradiation device (30) configured to direct a sterilizing irradiation onto the, in use, advancing web of packaging material (5).

9. Packaging machine according to claim 8, wherein the irradiation device (30) comprises one or more e-beam emitters (31, 32) for emitting electron beam irradiation onto the, in use, advancing web of packaging material (5).

10. Packaging machine according to any one of the preceding claims, wherein the sterilization apparatus (9) comprises a housing (33) having an inlet opening for receiving the web of packaging material (5) to be sterilized and an outlet opening for allowing feeding out of the sterilized web of packaging material (5);
wherein the outlet opening is adjacent to an inlet aperture of the isolation chamber (15) such that, in use, the web of packaging material (5) exits the sterilization apparatus (9) through the outlet opening and enters the isolation chamber (15) through the inlet aperture.

11. Packaging machine according to any one of the preceding claims, wherein the molding apparatus (7) comprises a plurality of molding units (11) configured to simultaneously mold molded portions (4) onto different sections of the web of packaging material (5).

12. Method for forming packages (2) filled with a pourable food product from a web of packaging material (5) ;
the method comprises the step of:
- advancing the web of packaging material (5) along a web advancement path (P);
- molding molded portions (4) onto the web of packaging material (5) at a molding station (8);
- sterilizing the web of packaging material (5) at a sterilization station (10), the sterilization station (10) being arranged downstream of the molding station (8) along the web advancement path (P);
- forming a tube (18) from the, in use, advancing web of packaging material (5) within an inner environment (16) of an isolation chamber (15);
- longitudinally sealing the tube (18) within the inner environment (16);
- filling the tube (18) with the pourable food product;
- producing the packages (2) from the tube (18) by forming, transversally sealing, and transversally cutting the tube (18); and
- preserving the cleanliness and/or sterility of at least the molded portions (4);
wherein during the step of preserving a flow of sterile fluid is directed against at least a portion of the web of packaging material (5) while advancing between the molding station (8) and the sterilization station (10) .

13. Method according to claim 12, wherein during the step of preserving the flow of sterile fluid is directed in a direction (D1) transversal to and/or opposite to an advancement direction (D2) of the web of packaging material (5).

14. Method according to claim 12 or 13, wherein during the step of preserving, a plurality of flows of sterile fluid are generated;
wherein each flow of sterile fluid is directed against a respective portion distinct from the other portions of the web of packaging material (5) while the web of packaging material (5) advances along a respective portion of the web advancement path (P) and while the web of packaging material (5) advances between the molding station (8) and the sterilization station (10).

15. Method according to any one of claims 12 to 14, wherein during the step of preserving, a sub-step of disinfecting is executed during which at least portions of the web of packaging material (5) are disinfected while advancing between the molding station (8) and the sterilization station (10).

## Patentansprüche

1. Verpackungsmaschine (1) zum Ausbilden von mit einem gießbaren Produkt gefüllten Verpackungen (2) aus einer Bahn aus Verpackungsmaterial (5);
wobei die Verpackungsmaschine (1) umfasst:
- eine Fördervorrichtung (6), die dazu ausgelegt ist, die Bahn aus Verpackungsmaterial (5) entlang eines Bahnvorschubpfads (P) vorzuschieben
- eine Formungseinrichtung (7), die an einer Formungsstation (8) zum Formen geformter Abschnitte (4) auf der Bahn aus Verpackungsmaterial (5) angeordnet ist;
- eine Sterilisationseinrichtung (9) zum Sterilisieren der Bahn aus Verpackungsmaterial (5) und die an einer Sterilisationsstation (10) entlang des Bahnvorschubpfads (P) stromabwärts der Formungsstation (8) angeordnet ist;
- eine Isolationskammer (15) mit einer Innenumgebung (16);
- eine Röhrenausbildungs-und -versiegelungsvorrichtung (17), die dazu ausgelegt ist, eine Röhre (18) aus der sich im Gebrauch vorschiebenden Bahn aus Verpackungsmaterial (5) innerhalb der Innenumgebung (16) an einer stromabwärts von angeordneten Röhrenausbildungsstation auszubilden und die Röhre (4) innerhalb der Innenumgebung in Längsrichtung zu versiegeln;
- eine Füllvorrichtung (20) zum Füllen der Röhre (18) mit dem gießbaren Produkt;
- eine Verpackungsausbildungseinrichtung (21), die dazu ausgelegt ist, die sich im Gebrauch vorschiebende Röhre (18) zum Ausbilden der Verpackungen (2) auszubilden, in Querrichtung zu versiegeln und in Querrichtung zu schneiden; und
- eine Konservierungsvorrichtung (22), die dazu ausgelegt ist, die Sauberkeit und/oder Sterilität zumindest der geformten Abschnitte (4) zu konservieren;
wobei die Konservierungsvorrichtung (22) mindestens eine Blaseinheit (39) umfasst, die dazu ausgelegt ist, einen Strom von sterilem Fluid gegen zumindest einen Abschnitt der Bahn aus Verpackungsmaterial (5) zu erzeugen, während sich die Bahn aus Verpackungsmaterial (5) im Gebrauch zwischen der Formungsstation (8) und der Sterilisationsstation (10) vorschiebt.

2. Verpackungsmaschine nach Anspruch 1, wobei die Blaseinheit (39) dazu ausgelegt ist, den Strom von sterilem Fluid in einer Richtung (D1) quer und/oder entgegengesetzt zu einer Vorschubrichtung (D2) der Bahn aus Verpackungsmaterial (5) zu erzeugen.

3. Verpackungsmaschine nach Anspruch 1 oder 2, wobei die Konservierungsvorrichtung (22) mehr als eine Blaseinheit (39) umfasst, von denen jede dazu ausgelegt ist, einen jeweiligen Strom von sterilem Fluid gegen zumindest einen jeweiligen Abschnitt der Bahn aus Verpackungsmaterial (5) zu erzeugen, während sich die Bahn aus Verpackungsmaterial (5) entlang eines jeweiligen Abschnitts des Bahnvorschubpfads (P) und zwischen der Formungsstation (8) und der Sterilisationsstation (10) vorschiebt.

4. Verpackungsmaschine nach einem der vorhergehenden Ansprüche, wobei die Konservierungsvorrichtung (22) auch eine Desinfektionseinheit zum Desinfizieren der Bahn aus Verpackungsmaterial (5), während sich die Bahn aus Verpackungsmaterial (5) zwischen der Formungsstation (8) und der Sterilisationsstation (10) vorschiebt, umfasst.

5. Verpackungsmaschine nach Anspruch 4, wobei die Desinfektionseinheit einen UV-Lichtemitter umfasst.

6. Verpackungsmaschine nach einem der vorhergehenden Ansprüche, und ferner umfassend ein Gehäuse, das einen Innenraum begrenzt, innerhalb dessen sich die Bahn aus Verpackungsmaterial (5) im Gebrauch vorschiebt, wenn sie sich entlang des Bahnvorschubpfads (P) bewegt.

7. Verpackungsmaschine nach Anspruch 6, wobei die Formungseinrichtung (7) innerhalb des Gehäuses angeordnet ist.

8. Verpackungsmaschine nach einem der vorhergehenden Ansprüche, wobei die Sterilisationseinrichtung (9) eine Bestrahlungsvorrichtung (30) umfasst, die dazu ausgelegt ist, eine Sterilisationsbestrahlung auf die sich im Gebrauch vorschiebende Bahn aus Verpackungsmaterial (5) zu richten.

9. Verpackungsmaschine nach Anspruch 8, wobei die Bestrahlungsvorrichtung (30) einen oder mehrere E-Strahl-Emitter (31, 32) zum Emittieren einer Elektronenstrahlbestrahlung auf die sich im Gebrauch vorschiebende Bahn aus Verpackungsmaterial (5) umfasst.

10. Verpackungsmaschine nach einem der vorhergehenden Ansprüche, wobei die Sterilisationseinrichtung (9) ein Gehäuse (33) umfasst, das eine Einlassöffnung zum Aufnehmen der zu sterilisierenden Bahn aus Verpackungsmaterial (5) und eine Auslassöffnung zum Ermöglichen eines Auslaufs der sterilisierten Bahn aus Verpackungsmaterial (5) aufweist;
wobei die Auslassöffnung an einen Einlassdurchgang der Isolationskammer (15) derart angrenzt, dass im Gebrauch die Bahn aus Verpackungsmaterial (5) durch die Auslassöffnung aus der Sterilisationseinrichtung (9) austritt und durch den Einlassdurchgang in die Isolationskammer (15) eintritt.

11. Verpackungsmaschine nach einem der vorhergehenden Ansprüche, wobei die Formungseinrichtung (7) eine Mehrzahl von Formungseinheiten (11) umfasst, die dazu ausgelegt sind, gleichzeitig geformte Abschnitte (4) auf unterschiedlichen Sektionen der Bahn aus Verpackungsmaterial (5) zu formen.

12. Verfahren zum Ausbilden von mit einem gießbaren Lebensmittelprodukt gefüllten Verpackungen (2) aus einer Bahn aus Verpackungsmaterial (5);
wobei das Verfahren den folgenden Schritt umfasst:
- Vorschieben der Bahn aus Verpackungsmaterial (5) entlang eines Bahnvorschubpfads (P);
- Formen von geformten Abschnitten (4) auf der Bahn aus Verpackungsmaterial (5) an einer Formungsstation (8);
- Sterilisieren der Bahn aus Verpackungsmaterial (5) an einer Sterilisationsstation (10), wobei die Sterilisationsstation (10) entlang des Bahnvorschubpfads (P) stromabwärts der Formungsstation (8) angeordnet ist;
- Ausbilden einer Röhre (18) aus der sich im Gebrauch vorschiebenden Bahn aus Verpackungsmaterial (5) innerhalb einer Innenumgebung (16) einer Isolationskammer (15);
- Versiegeln in Längsrichtung der Röhre (18) innerhalb der Innenumgebung (16);
- Füllen der Röhre (18) mit dem gießbaren Lebensmittelprodukt;
- Herstellen der Verpackungen (2) aus der Röhre (18) durch Ausbilden, Versiegeln in Querrichtung und Schneiden in Querrichtung der Röhre (18); und
- Konservieren der Sauberkeit und/oder Sterilität zumindest der geformten Abschnitte (4);
wobei während des Schritts zum Konservieren ein Strom von sterilem Fluid gegen zumindest einen Abschnitt der Bahn aus Verpackungsmaterial (5) während eines Vorschiebens zwischen der Formungsstation (8) und der Sterilisationsstation (10) gerichtet wird.

13. Verfahren nach Anspruch 12, wobei während des Schritts zum Konservieren der Strom von sterilem Fluid in eine Richtung (D1) quer zu und/oder entgegengesetzt zu einer Vorschubrichtung (D2) der Bahn aus Verpackungsmaterial (5) gerichtet wird.

14. Verfahren nach Anspruch 12 oder 13, wobei während des Schritts zum Konservieren eine Mehrzahl von Strömen von sterilem Fluid erzeugt werden;
wobei jeder Strom von sterilem Fluid gegen einen jeweiligen Abschnitt gerichtet wird, der sich von den anderen Abschnitten der Bahn aus Verpackungsmaterial (5) unterscheidet, während sich die Bahn aus Verpackungsmaterial (5) entlang eines jeweiligen Abschnitts des Bahnvorschubpfads (P) vorschiebt und während sich die Bahn aus Verpackungsmaterial (5) zwischen der Formungsstation (8) und der Sterilisationsstation (10) vorschiebt.

15. Verfahren nach einem der Ansprüche 12 bis 14, wobei während des Schritts zum Konservieren ein Teilschritt zum Desinfizieren ausgeführt wird, während dessen zumindest Abschnitte der Bahn aus Verpackungsmaterial (5) während eines Vorschiebens zwischen der Formungsstation (8) und der Sterilisationsstation (10) desinfiziert werden.

## Revendications

1. Machine d'emballage (1) destinée à former des emballages (2) remplis d'un produit fluide à partir d'une bande de matériau d'emballage (5) ;
la machine d'emballage (1) comprenant :
un dispositif de transport (6) conçu pour faire avancer la bande de matériau d'emballage (5) le long d'un chemin d'avancement de bande (P) ;
un appareil de moulage (7) disposé au niveau d'une station de moulage (8) pour mouler des parties moulées (4) sur la bande de matériau d'emballage (5) ;
un appareil de stérilisation (9) destiné à stériliser la bande de matériau d'emballage (5) et disposé au niveau d'une station de stérilisation (10) en aval de la station de moulage (8) le long du chemin d'avancement de bande (P) ;
une chambre d'isolement (15) ayant un environnement intérieur (16) ;
un dispositif de formation et de scellement de tube (17) conçu pour former un tube (18) à partir de la bande de matériau d'emballage (5) qui avance, lors de l'utilisation, dans l'environnement intérieur (16) au niveau d'une station de formation de tube située en aval de et pour sceller longitudinalement le tube (4) à l'intérieur de l'environnement intérieur ;
un dispositif de remplissage (20) destiné à remplir le tube (18) avec le produit fluide ;
un appareil de formation d'emballage (21) conçu pour former, sceller transversalement et couper transversalement le tube (18) qui avance, lors de l'utilisation, pour former les emballages (2) ; et
un dispositif de préservation (22) conçu pour préserver la propreté et/ou la stérilité d'au moins les parties moulées (4) ;
le dispositif de préservation (22) comprenant au moins une unité de soufflage (39) conçue pour générer un flux de fluide stérile contre au moins une partie de la bande de matériau d'emballage (5) pendant que la bande de matériau d'emballage (5) avance, lors de l'utilisation, entre la station de moulage (8) et la station de stérilisation (10).

2. Machine d'emballage selon la revendication 1, l'unité de soufflage (39) étant conçue pour générer le flux de fluide stérile dans une direction (D1) transversale et/ou opposée à une direction d'avancement (D2) de la bande de matériau d'emballage (5).

3. Machine d'emballage selon la revendication 1 ou 2, le dispositif de préservation (22) comprenant plus d'une unité de soufflage (39), chacune étant conçue pour générer un flux respectif de fluide stérile contre au moins une partie respective de la bande de matériau d'emballage (5) pendant que la bande de matériau d'emballage (5) avance le long d'une partie respective du chemin d'avancement de bande (P) et entre la station de moulage (8) et la station de stérilisation (10).

4. Machine d'emballage selon l'une quelconque des revendications précédentes, le dispositif de préservation (22) comprenant également une unité de désinfection pour désinfecter la bande de matériau d'emballage (5) pendant que la bande de matériau d'emballage (5) avance entre la station de moulage (8) et la station de stérilisation (10).

5. Machine d'emballage selon la revendication 4, l'unité de désinfection comprenant un émetteur de lumière UV.

6. Machine d'emballage selon l'une quelconque des revendications précédentes, comprenant en outre un boîtier délimitant un espace intérieur à l'intérieur duquel la bande de matériau d'emballage (5) avance, lors de l'utilisation, lorsqu'elle se déplace le long du chemin d'avancement de bande (P).

7. Machine d'emballage selon la revendication 6, l'appareil de moulage (7) étant disposé à l'intérieur du boîtier.

8. Machine d'emballage selon l'une quelconque des revendications précédentes, l'appareil de stérilisation (9) comprenant un dispositif de rayonnement (30) conçu pour diriger un rayonnement stérilisant sur la bande de matériau d'emballage (5) qui avance, lors de l'utilisation.

9. Machine d'emballage selon la revendication 8, le dispositif de rayonnement (30) comprenant un ou plusieurs émetteurs de faisceaux d'électrons (31, 32) pour émettre des rayons de faisceaux d'électrons sur la bande de matériau d'emballage (5) qui avance, lors de l'utilisation.

10. Machine d'emballage selon l'une quelconque des revendications précédentes, l'appareil de stérilisation (9) comprenant un boîtier (33) ayant une ouverture d'entrée pour recevoir la bande de matériau d'emballage (5) à stériliser et une ouverture de sortie pour permettre l'évacuation de la bande stérilisée de matériau d'emballage (5) ;
l'ouverture de sortie étant adjacente à une ouverture d'entrée de la chambre d'isolement (15) de sorte que, lors de l'utilisation, la bande de matériau d'emballage (5) sorte de l'appareil de stérilisation (9) par l'ouverture de sortie et entre dans la chambre d'isolement (15) par l'ouverture d'entrée.

11. Machine d'emballage selon l'une quelconque des revendications précédentes, le dispositif de moulage (7) comprenant une pluralité d'unités de moulage (11) conçues pour mouler simultanément des parties moulées (4) sur différentes sections de la bande de matériau d'emballage (5) .

12. Procédé de formation d'emballages (2) remplis d'un produit alimentaire fluide à partir d'une bande de matériau d'emballage (5) ;
le procédé comprend les étapes consistant à :
faire avancer la bande de matériau d'emballage (5) le long d'un chemin d'avancement de bande (P) ;
mouler des parties moulées (4) sur la bande de matériau d'emballage (5) au niveau d'une station de moulage (8) ;
stériliser la bande de matériau d'emballage (5) au niveau d'une station de stérilisation (10), la station de stérilisation (10) étant disposée en aval de la station de moulage (8) le long du chemin d'avancement de bande (P) ;
former un tube (18) à partir de la bande de matériau d'emballage (5) qui avance, lors de l'utilisation, dans un environnement intérieur (16) d'une chambre d'isolement (15) ;
sceller longitudinalement le tube (18) dans l'environnement intérieur (16) ;
remplir le tube (18) avec le produit alimentaire fluide ;
produire les emballages (2) à partir du tube (18) en formant, scellant transversalement et coupant transversalement le tube (18) ; et
préserver la propreté et/ou la stérilité d'au moins les parties moulées (4) ;
au cours de l'étape de préservation, un flux de liquide stérile étant dirigé contre au moins une partie de la bande de matériau d'emballage (5) pendant qu'elle avance entre la station de moulage (8) et la station de stérilisation (10).

13. Procédé selon la revendication 12, au cours de l'étape de préservation, le flux de fluide stérile étant dirigé dans une direction (D1) transversale et/ou opposée à une direction d'avancement (D2) de la bande de matériau d'emballage (5).

14. Procédé selon la revendication 12 ou 13, pendant l'étape de préservation, une pluralité de flux de liquide stérile étant générés ;
chaque flux de fluide stérile étant dirigé contre une partie respective distincte des autres parties de la bande de matériau d'emballage (5) pendant que la bande de matériau d'emballage (5) avance le long d'une partie respective du chemin d'avancement de bande (P) et pendant que la bande de matériau d'emballage (5) avance entre la station de moulage (8) et la station de stérilisation (10) .

15. Procédé selon l'une quelconque des revendications 12 à 14, pendant l'étape de préservation, une sous-étape de désinfection étant exécutée au cours de laquelle au moins des parties de la bande de matériau d'emballage (5) sont désinfectées tout en avançant entre la station de moulage (8) et la station de stérilisation (10).
